# EUROPÄISCHE PATENTSCHRIFT

(11) **EP 1 311 303 B1**
(45) Veröffentlichungstag und Bekanntmachung des Hinweises auf die Patenterteilung: **13.06.2007**
(21) Anmeldenummer: 01958058.8
(22) Anmeldetag: 07.08.2001
(51) Int. Cl.: A61K 51/12, A61K 51/02, A61P 29/00

(54) **NEUE, 224 RA ENTHALTENDE, RADIOTHERAPEUTISCH WIRKSAME FORMULIERUNGEN UND VERFAHREN ZU IHRER HERSTELLUNG**
NOVEL RADIOTHERAPEUTIC FORMULATIONS CONTAINING 224 RA AND A METHOD FOR THEIR PRODUCTION
NOUVELLES FORMULATIONS D'ACTION RADIOTHERAPEUTIQUE ET CONTENANT DU 224 RA ET LEURS PROCEDES DE PRODUCTION

(30) Priorität: 21.08.2000 DE 10040771
(43) Veröffentlichungstag der Anmeldung: 21.05.2003
(73) Patentinhaber: Altmann Therapie GmbH & Co. KG, 38229 Salzgitter (DE)
(72) Erfinder: SCHWARZ, Uwe, 38228 Salzgitter (DE); DANIELS, Rolf, 38226 Salzgitter (DE)
(74) Vertreter: Savic, Bojan
(86) Internationale Anmeldenummer: PCT/EP2001/009121
(87) Internationale Veröffentlichungsnummer: WO 2002/015943

(56) Entgegenhaltungen:
- US-A- 4 663 129
- US-A- 5 457 323

## Beschreibung

Die vorliegende Erfindung betrifft neue, ²²⁴Ra enthaltende, radiotherapeutisch wirksame Formulierungen und Verfahren zu ihrer Herstellung.

Anklylosing Spondylitis (*Morbus Bechterew*) ist ein mit degenerativen Ossifikationsprozessen der Wirbelsäure und bleibender Rückgratverkrümmung einhergehendes, seit langem bekanntes Krankheitsbild. Die Verwendung von Formulierungen auf der Basis von Salzen des Radioisotops ²²⁴Ra zur Therapie dieser Erkrankung ist ebenfalls seit Jahrzehnten bekannter Stand der Technik. So beschreibt *Orhan Delikan* in der Literaturstelle Health Physics, Vol. 35(July), pp. 21 -24, Pergamon Press Ltd., 1978 daß für den Erfolg oder Mißerfolg einer derartigen Therapie der Begrenzung des Gehalts an verunreinigenden Radiosotopen, so wie zum Beispiel den besonders langlebigen, α-Teilchen emittierenden Nukliden ²²⁸Th, ²²⁶Ra, ²²⁸Ra oder ²²⁷Ac, eine entscheidende Bedeutung zukommt. Derartige Verunreinigungen werden aufgrund ihrer ausgeprägten strukturchemischen Isomorphie mit dem Isotop ²²⁴Ra leicht vom Patienten in die Knochensubstanz eingebaut und dort akkumuliert, was die eigentliche Radiotherapie konterkariert oder zumindest mit erheblichen, für den Patienten belastenden Nebenwirkungen verbunden ist. Da die Herstellung des therapeutisch wirksamen Isotops ²²⁴Ra ausgehend vom Isotop ²²⁸Th unter Emission eines α-Teilchens (⁴He) erfolgt, kommt sowohl der vorausgehenden Reinigung des eingesetzten Ausgangsmaterials ²²⁸Th, als auch einer anschließenden, möglichst gründlichen Abtrennung des erhaltenen Produktisotops ²²⁴Ra aus dem Reaktionsgemisch im Hinblick auf die erwünschte Herstellung eines möglichst wirksamen Therapeutikums ohne unerwünschte Randeffekte und Nebenwirkungen eine entscheidende Bedeutung zu.

Entsprechend dem Stand der Technik wird die Aufreinigung des Ausgangsmaterials ²²⁸Th durch ionenaustauschchromatographische Abtrennung der vorstehend erwähnten, störenden Isotope vorgenommen. Anschließend erfolgt die Fällung des ²²⁸Th als schwerlösliches Hydroxid und das Waschen des Sediments. Nach Neubildung des Tochterisotops ²²⁴Ra erfolgt im Verlauf von ca. 5 bis 10 Tagen dann die Extraktion des als Hydroxid in wäßriger Lösung vorliegenden ²²⁸Th mit Hilfe einer Calciumchloridlösung, was nach mehrmaliger Wiederholung zu einer mehr oder weniger reinen, wäßrigen (²²⁴Ra) RaCl₂-Lösung führt.

Beide vorstehend beschriebenen Reinigungsschritte werden jedoch von einschlägig vorgebildeten Fachleuten als äußerst unbefriedigend beschrieben. So vermerkt die erwähnte Literaturstelle von *Delikan* im die Seiten 22 und 23 verbindenden Absatz, dass die ²²⁸ Th-Extraktion "sehr kritisch" ist, da hierbei verschiedene, schwierig einzustellende Randbedingungen und Verfahrensparameter gleichzeitig beachtet werden müssen, deren Einhaltung die wirksame Durchführung dieses Reinigungsschrittes auch dem bestens ausgebildeten und ausgerüsteten Fachmann nachhaltig erschwert, bzw. verunmöglicht. Auf Seite 23, linke Spalte, wird zu Beginn des ersten, vollständigen Absatzes zudem vermerkt, daß nach derartigen Verfahren des Standes der Technik hergestellte, ²²⁴Ra enthaltende Formulierungen regelmäßig trotz aller Reinigungsschritte noch einen meßbaren Restgehalt an den oben erwähnten, langlebigen, Radionukliden enthalten, was darauf zurückgeführt werden kann, daß die Salze dieser Nuklide nicht vollständig unlöslich sind und somit zum Teil auch nach der Extraktion des ²²⁸ Th in der therapeutisch verwendeten ²²⁴Ra-Formulierung verbleiben.

Es besteht somit entsprechend dem Stand der Technik ein Bedarf an einer radiotherapeutisch wirksamen Formulierung zur Therapie von Anklylosing Spondylitis (*= Morbus Bechterew*)*,* welche die oben beschriebenen Nachteile der Formulierungen des Standes der Technik durch einen gegenüber den bekannten Formulierungen höheren Reinheitsgrad à priori vermeidet, insbesondere durch einen wesentlich niedrigeren Gehalt an den oben beschriebenen, langlebigen Nukliden ²²⁸Th, ²²⁶Ra, ²²⁸Ra oder ²²⁷Ac.

Es wurde nun überraschend gefunden, dass diese Aufgabe durch eine neuartige, das Isotop ²²⁴Ra enthaltende, therapeutisch wirksame Formulierung gelöst wird.

Diese erfindungsgemäße Formulierung umfasst mindestens ein Salz des Isotops ²²⁴Ra und ist dadurch gekennzeichnet, dass der Gehalt an weiteren Radionukliden, insbesondere der Gehalt an den nachfolgend aufgeführten Radionukliden jeweils nicht über den angegebenen Zahlenwert hinausgeht:

| Radionuklid: | max. Konzentration (mBq / g) |
|---|---|
| ²²⁸Ra | 60 |
| ²²⁸Th | 30 |
| ²¹⁰Pb | 30 |
| ²²⁶Ra | 30 |
| ²³⁸U | 20 |
| ²³¹Pa | 60 |
| ²²⁷Ac | 10 |
| ²³²U | 50. |

Gemäß einer bevorzugten Ausführungsform ist die erfindungsgemäße Formulierung dadurch gekennzeichnet, dass der Gehalt an weiteren Radionukliden, insbesondere der Gehalt an den nachfolgend aufgeführten Radionukliden jeweils nicht über den angegebenen Zahlenwert hinausgeht:

| Radionuklid: | max. Konzentration (mBq / g) |
|---|---|
| ²²⁸Ra | 26 |
| ²²⁸Th | 3,6 |
| ²¹⁰Pb | 23 |
| ²²⁶Ra | 29 |
| ²³⁸U | 19 |
| ²³¹Pa | 55 |
| ²²⁷Ac | 9,3 |
| ²³²U | 20. |

Gemäß einer besonders bevorzugten Ausführungsform ist die erfindungsgemäße Formulierung dadurch gekennzeichnet, dass der Gehalt an weiteren Radionukliden,
insbesondere der Gehalt an den nachfolgend aufgeführten Radionukliden jeweils nicht über den angegebenen Zahlenwert hinausgeht:

| Radionuklid: | max. Konzentration (mBq / g) |
|---|---|
| ²²⁸Ra | 22 |
| ²²⁸Th | 3,3 |
| ²¹⁰Pb | 17 |
| ²²⁶Ra | 26 |
| ²³⁸U | 15 |
| ²³¹Pa | 55 |
| ²²⁷Ac | 8,4 |
| ²³²U | 5. |

Gemäß einer weiteren, bevorzugten Ausführungsform ist die erfindungsgemäße Formulierung dadurch gekennzeichnet, dass es sich bei dem Salz des Radionuklids ²²⁴Ra um ²²⁴Radiumchlorid (²²⁴RaCl₂) handelt. Außerdem umfasst die erfindungsgemäße Formulierung vorzugsweise ein Isotonikum, besonders bevorzugt ein Calciumsalz, insbesondere Calciumchlorid.

Die erfindungsgemäße Formulierung wird mit Hilfe eines Verfahrens hergestellt, welches dadurch gekennzeichnet ist, dass die nachfolgend beschriebenen Verfahrensschritte (a) bis (d) durchgeführt werden:
(a) Mindestens einmalige Zentrifugation einer wäßrigen Suspension der Verbindung ²²⁸ Th(OH)₄, gegebenenfalls nach Resuspendierung des erhaltenen Rohsediments;
(b) Abtrennung des nach Schritt (a) ausgefällten ²²⁸ Th(OH)₄-Sediments;
(c) Sterilfiltration und anschließende Abfüllung der erwünschten Dosis der nach Schritt (b) erhaltenen, überstehenden Lösung eines ²²⁴Ra-Salzes;
(d) Sterilisation der nach Schritt (c) erhaltenen Abfüllung in an sich bekannter Weise.

Eine bevorzugte Ausführungsform des erfindungsgemäßen Verfahrens ist dadurch gekennzeichnet, dass man die Zentrifugation in Schritt (a) zweimal durchführt und den erhaltenen Niederschlag danach jeweils mit Hilfe einer wäßrigen Lösung eines Isotonikums, besonders bevorzugt eines Calciumsalzes, insbesondere einer 1,14 Gew.-prozentigen CaCl₂-Lösung resuspendiert.

Eine andere bevorzugte Ausführungsform des erfindungsgemäßen Verfahrens ist dadurch gekennzeichnet, dass man die Zentrifugation in Schritt (a) mit einer Rotationsgeschwindigkeit entsprechend einer Zentrifugalbeschleunigung von 2000 g während eines Zeitraumes von 10 Minuten durchführt.

Eine weitere bevorzugte Ausführungsform des erfindungsgemäßen Verfahrens ist dadurch gekennzeichnet, dass man die Abtrennung in Schritt (b) durch Absaugen und Filtrieren durchführt.

Eine weitere bevorzugte Ausführungsform des erfindungsgemäßen Verfahrens ist dadurch gekennzeichnet; dass man die Sterilfiltration in Schritt (c) unter Verwendung eines bekannten Sterilfiltermaterials durchführt, besonders bevorzugt unter Verwendung eines Materials auf der Basis eines fluorhaltigen, organischen Gebrauchspolymers oder eines gegebenenfalls modifizierten und/oder derivatisierten Polysaccharids.

Eine besonders bevorzugte Ausführungsform des erfindungsgemäßen Verfahrens ist dadurch gekennzeichnet, dass das Sterilfiltermaterial Zellulose, Zelluloseacetat oder Polytetrafluorethylen, besonders bevorzugt Zelluloseacetat ist.

Eine bevorzugte Ausführungsform des erfindungsgemäßen Verfahrens ist dadurch gekennzeichnet, dass das Sterilfiltermaterial eine Porengröße im Bereich von etwa 0,01 µm bis 10 µm aufweist, besonders bevorzugt im Bereich von etwa 0,2 µm bis 5 µm und insbesondere von etwa 0,2 µm.

Eine weitere bevorzugte Ausführungsform des erfindungsgemäßen Verfahrens ist dadurch gekennzeichnet, dass man den Sterilisationsschritt (d) unter Verwendung eines üblichen Autoklaven durchführt, besonders bevorzugt bei einer Temperatur von etwa 121 °C während eines Zeitraums von etwa 20 Minuten.

Eine weitere bevorzugte Ausführungsform des erfindungsgemäßen Verfahrens ist dadurch gekennzeichnet, dass es sich bei dem therapeutisch wirksamen Salz des Radionuklids ²²⁴Ra um ²²⁴Radiumchlorid (²²⁴RaCl₂) handelt.

Die vorliegende Erfindung wird in den folgenden Textabschnitten anhand entsprechender, repräsentativer Ausführungsbeispiele ausführlicher beschrieben.

### Ausführungsbeispiele

### 1. Herstellung von [²²⁸Th] Thoriumhydroxid

### Herstellung von ²²⁸Th

Die Herstellung von ²²⁸Th erfolgt ausgehend von ²³¹Pa durch die ²³¹Pa(n y)²³²Pa Reaktion. Das bei dieser Reaktion entstandene ²³²Pa zerfällt erst ZU ²³²U und dann zu ²²⁸Th. Für die Herstellung wurde Protactiniumpentoxid von seinen Töchtern gereinigt, in Quarzvials eingeschmolzen und in einem Reaktor bestrahlt. Nach einer Abklingzeit von einigen Wochen erfolgte eine ionenaustauschchromatographische Abtrennung des ²³²U (t 1/2 = 72 Jahre), dass dann als Chlorid aufgefangen, getrocknet und gelagert wurde, um das ²²⁸Th zu erhalten.

Dieses Material wurde in HNO₃ gelöst und über eine Anionenaustauschersäule gegeben. Unter diesen Bedingungen wird Thorium als [Th(NO₃)₆]²⁻ an der Säule gebunden und die Töchter des ²²⁸Th sowie ²³²U mit HNO₃ von der Säule gewaschen. Anschließend wurde ²²⁸Th mit einer 0,5 m HCI von der Säule gewaschen. Nach einer Konzentrationsbestimmung wurde diese Lösung in Glasgefäße überführt und getrocknet.

### Herstellung von [^{228Th}] Thoriumhydroxid

1. Das [²²⁸Th] Thoriumchlorid wird mit Hilfe der Trägerlösung ([²³² Th] Thoriumnitrat Pentahydrat in Salzsäure 10%, Ph. Eur.*) in Teilschritten im Primärbehältnis aufgenommen und in ein 50 ml - Zentrifugenglas überführt.
2. Aus dieser Lösung wird mit Ammoniaklösung (26%) das unlösliche Thoriumhydroxid gefällt. Mit Calciumchloridlösung wird auf 30 ml aufgefüllt und bei 2000 g während 10 Minuten zentrifugiert. Die Zugabe von (²³² Th] Thoriumnitrat Pentahydrat als Träger in Schritt 1 garantiert die Entstehung von ausreichend großen Niederschlagsmengen, die sich durch Zentrifugation quantitativ abtrennen lassen, und minimiert Adsorptionseffekte des ²²⁸Th.
3. Der Überstand wird durch eine Glasfilternutsche vorsichtig abgesaugt. Der Rückstand wird mit Calciumchloridlösung 1,14% versetzt und nach Aufrühren mit einem Magnetrührer erneut zentrifugiert. Dieser Waschschritt wird noch einmal wiederholt, um eventuell vorhandene nicht sedimentierbare Thoriumhydroxid Spuren vollständig zu entfernen. Beim letzten Absaugen wird möglichst der gesamte Überstand entfernt.
4. Das so erhaltene Rohsediment wird getrocknet.
5. Das getrocknete Rohsediment wird erneut in Calciumchloridlösung 1,14% suspendiert und im verschlossenen Zentrifugenglas aufbewahrt.

### 2. Herstellung von [²²⁴ Ra] Radiumchlorid

Die Herstellung von [²²⁴ Ra] Radiumchlorid erfolgt in 4 Stufen. Die Stufen 1-3 werden in einer gasdichten Box mit Bleiabschirmung und Fernbedienung ausgeführt. Die Zuluft wird durch Schwebstoffilter der Klasse S steril gefiltert. Benötigte Gegenstände können über Schleusen in die Box gebracht werden.

### Stufe 1 Extraktion und Zentrifugation

Das lösliche [²²⁴ Ra] Radiumchlorid wird mit Calciumchloridlösung vom unlöslichen [²²⁸Th] Thoriumhydroxid desorbiert und durch Zentrifugieren abgetrennt.
Dieser Schritt wird mehrfach durchgeführt. Die Überstände werden nach jeder Zentrifugation abgesaugt und anschließend zur Chargenstammlösung vereinigt. Die Herstellungssuspension wird resuspendiert und gelagert.

### Stufe 2 Dosis-Abfüllung in Injektionsflaschen

Zur Bestimmung der [²²⁴ Ra] Radiumchlorid Konzentration erfolgt eine Aktivitätskontrollmessung eines Aliquotes der Chargenstammlösung.
In die Injektionsflaschen einer typischen Charge werden aufgrund der ermittelten Aktivitätskonzentration Chargenstammlösung und entsprechend Calciumchloridlösung (1,14%) mit Hilfe eines Zweispritzen - Dilutors mit sterilen Spritzenvorsatzfiltern abgefüllt. Wird für mehrere Kalibrierungszeitpunkte am gleichen Tag abgefüllt, wird die Menge der zuzugebenden Chargenstammlösung entsprechend dem Zerfall von Radium-224 (T _{1/2 phys} = 3,66 Tage) geändert und die Menge der Calciumchloridlösung entsprechend auf 1,1 ml ergänzt. Die Flaschen werden dann verschlossen und versiegelt.

### Stufe 3 Sterilisation

Das Präparat wird bei 121 °C über 20 Minuten in einem Laborautoklaven sterilisiert.

### Stufe 4 Etikettieren der äußeren Behältnisse und Verpacken

Nach Sterilisation werden die Flaschen, welche bereits vor Abfüllung etikettiert waren, aus der Produktionsbox ausgerührt und verpackt.

## Patentansprüche

1. Radiotherapeutisch wirksame Formulierung, welche mindestens ein Salz des Isotops ²²⁴Ra umfasst und welche **dadurch gekennzeichnet ist, dass** der Gehalt an weiteren Radionukliden, insbesondere der Gehalt an den nachfolgend aufgeführten Radionukliden jeweils nicht über den angegebenen Zahlenwert hinausgeht:
| Radionuklid: | max. Konzentration (mBq / g) |
|---|---|
| ²²⁸Ra | 60 |
| ²²⁸Th | 30 |
| ²¹⁰Pb | 30 |
| ²²⁶Ra | 30 |
| ²³⁸U | 20 |
| ²³¹Pa | 60 |
| ²²⁷Ac | 10 |
| ²³²U | 50, |
hergestellt durch ein Verfahren, umfassend die nachfolgend beschriebenen Verfahrensschritte (a) bis (d):
(a) Mindestens einmalige Zentrifugation einer wäßrigen Suspension der Verbindung ²²⁸ Th(OH)₄, gegebenenfalls nach Resuspendierung des erhaltenen Rohsediments;
(b) Abtrennung des nach Schritt (a) ausgefällten ²²⁸ Th(OH)₄-Sediments;
(c) Sterilfiltration und anschließende Abfüllung der erwünschten Dosis der nach Schritt (b) erhaltenen, überstehenden Lösung eines ²²⁴ Ra-Salzes;
(d) Sterilisation der nach Schritt (c) erhaltenen Abfüllung in an sich bekannter Weise.

2. Formulierung nach Anspruch 1, **dadurch gekennzeichnet, dass** der Gehalt an weiteren Radionukliden, insbesondere der Gehalt an den nachfolgend aufgeführten Radionukliden jeweils nicht über den angegebenen Zahlenwert hinausgeht:
| Radionuklid: | max. Konzentration (mBq / g) |
|---|---|
| ²²⁸Ra | 26 |
| ²²⁸Th | 3,6 |
| ²¹⁰Pb | 23 |
| ²²⁶Ra | 29 |
| ²³⁸U | 19 |
| ²³¹Pa | 55 |
| ²²⁷Ac | 9,3 |
| ²³²U | 20, |
hergestellt durch ein Verfahren, umfassend die nachfolgend beschriebenen Verfahrensschritte (a) bis (d):
(a) Mindestens einmalige Zentrifugation einer wäßrigen Suspension der Verbindung ²²⁸ Th(OH)₄, gegebenenfalls nach Resuspendierung des erhaltenen Rohsediments;
(b) Abtrennung des nach Schritt (a) ausgefällten ²²⁸ Th(OH)₄-Sediments;
(c) Sterilfiltration und anschließende Abfüllung der erwünschten Dosis der nach Schritt (b) erhaltenen, überstehenden Lösung eines ²²⁴Ra-Salzes;
(d) Sterilisation der nach Schritt (c) erhaltenen Abfüllung in an sich bekannter Weise.

3. Formulierung nach Anspruch 1, **dadurch gekennzeichnet, dass** der Gehalt an weiteren Radionukliden, insbesondere der Gehalt an den nachfolgend aufgeführten Radionukliden jeweils nicht über den angegebenen Zahlenwert hinausgeht:
| Radionuklid: | max. Konzentration (mBq / g) |
|---|---|
| ²²⁸Ra | 22 |
| ²²⁸Th | 3,3 |
| ²¹¹Pb | 17 |
| ²²⁶Ra | 26 |
| ²³⁸U | 15 |
| ²³¹Pa | 55 |
| ²²⁷Ac | 8,4 |
| ²³²U | 5, |
hergestellt durch ein Verfahren, umfassend die nachfolgend beschriebenen Verfahrensschritte (a) bis (d):
(a) Mindestens einmalige Zentrifugation einer wäßrigen Suspension der Verbindung ²²⁸ Th(OH)₄, gegebenenfalls nach Resuspendierung des erhaltenen Rohsediments;
(b) Abtrennung des nach Schritt (a) ausgefällten ²²⁸ Th(OH)₄-Sediments;
(c) Sterilfiltration und anschließende Abfüllung der erwünschten Dosis der nach Schritt (b) erhaltenen, überstehenden Lösung eines ²²⁴Ra-Salzes;
(d) Sterilisation der nach Schritt (c) erhaltenen Abfüllung in an sich bekannter Weise.

4. Formulierung nach einem der Ansprüche 1 bis 3, **dadurch gekennzeichnet, dass** es sich bei dem Salz des Radionuklids ²²⁴Ra um ²²⁴Radiumchlorid (²²⁴RaCl₂) handelt.

5. Formulierung nach einem der Ansprüche 1 bis 4, **dadurch gekennzeichnet, dass** die Formulierung ein Isotonikum, vorzugsweise ein Calciumsalz, insbesondere Calciumchlorid umfasst.

6. Verfahren zur Herstellung einer radiotherapeutisch wirksamen Formulierung, welche mindestens ein Salz des Isotops ²²⁴Ra umfasst, **dadurch gekennzeichnet, dass** die nachfolgend beschriebenen Verfahrensschritte (a) bis (d) durchgeführt werden:
(a) Mindestens einmalige Zentrifugation einer wäßrigen Suspension der Verbindung ²²⁸ Th(OH)₄, gegebenenfalls nach Resuspendierung des erhaltenen Rohsediments;
(b) Abtrennung des nach Schritt (a) ausgefällten ²²⁸ Th(OH)₄-Sediments;
(c) Sterilfiltration und anschließende Abfüllung der erwünschten Dosis der nach Schritt (b) erhaltenen, überstehenden Lösung eines ²²⁴Ra-Salzes;
(d) Sterilisation der nach Schritt (c) erhaltenen Abfüllung in an sich bekannter Weise.

7. Verfahren nach Anspruch 6, **dadurch gekennzeichnet, dass** man die Zentrifugation in Schritt (a) zweimal durchführt und den erhaltenen Niederschlag danach jeweils mit Hilfe einer wäßrigen Lösung eines Isotonikums, vorzugsweise eines Calciumsalzes, insbesondere einer 1,14 Gew.-prozentigen CaCl₂-Lösung resuspendiert.

8. Verfahren nach einem der Ansprüche 6 oder 7; **dadurch gekennzeichnet, dass** man die Zentrifugation in Schritt (a) mit einer Rotationsgeschwindigkeit entsprechend einer Zentrifugalbeschleunigung von 2000 g während eines Zeitraumes von 10 Minuten durchführt.

9. Verfahren nach einem der Ansprüche 6 bis 8, **dadurch gekennzeichnet, dass** man die Abtrennung in Schritt (b) durch Absaugen und Filtrieren durchführt.

10. Verfahren nach einem der Ansprüche 6 bis 9, **dadurch gekennzeichnet, dass** man die Sterilfiltration in Schritt (c) unter Verwendung eines bekannten Sterilfiltermaterials durchführt, vorzugsweise unter Verwendung eines Materials auf der Basis eines fluorhaltigen, organischen Gebrauchspolymers oder eines gegebenenfalls modifizierten und/oder derivatisierten Polysaccharids.

11. Verfahren nach einem der Ansprüche 6 bis 10, **dadurch gekennzeichnet, dass** das Sterilfiltermaterial Zellulose, Zelluloseacetat oder Polytetrafluorethylen, vorzugsweise Zelluloseacetat ist.

12. Verfahren nach einem der Ansprüche 6 bis 11, **dadurch gekennzeichnet, dass** das Sterilfiltermaterial eine Porengröße im Bereich von etwa 0,01 µm bis 10 µm aufweist, vorzugsweise im Bereich von etwa 0,2 µm bis 5 µm und insbesondere von etwa 0,2 µm.

13. Verfahren nach einem der Ansprüche 6 bis 12, **dadurch gekennzeichnet, dass** man den Sterilisationsschritt (d) unter Verwendung eines üblichen Autoklaven durchführt, vorzugsweise bei einer Temperatur von etwa 121 °C während eines Zeitraums von etwa 20 Minuten.

14. Verfahren nach einem der Ansprüche 6 bis 13, **dadurch gekennzeichnet, dass** es sich bei dem therapeutisch wirksamen Salz des Radionuklids ²²⁴Ra um ²²⁴Radiumchlorid (²²⁴RaCl₂) handelt.

15. Verwendung einer Formulierung gemäß einem der Ansprüche 1 bis 5 zur Herstellung eines Arzneimittels zur Therapie von Anklylosing Spondylitis (*Morbus Bechterew*)*.*

## Claims

1. Radiotherapeutic effective formulation which comprises at least one salt of the isotope ²²⁴Ra and which is **characterized in that** the content of other radionuclides, especially the content of the radionuclides cited below, does not exceed the indicated numerical value for each:
| radionuclide | max. concentration (mBq/g) |
|---|---|
| ²²⁸Ra | 60 |
| ²²⁸Th | 30 |
| ²¹⁰Pb | 30 |
| ²²⁶Ra | 30 |
| ²³⁸U | 20 |
| ²³¹Pa | 60 |
| ²²⁷Ac | 10 |
| ²³²U | 50 |
produced by a process which comprises the process steps (a) to (d) described below:
(a) at least one-time centrifugation of an aqueous suspension of the compound ²²⁸Th(OH)₄, optionally after resuspension of the resulting raw sediment;
(b) separation of the ²²⁸Th(OH)₄ sediment which was precipitated according to step (a);
(c) sterile filtration and subsequent bottling of the desired dose of the supernatant solution of the ²²⁴Ra salt which was obtained according to step (b);
(d) sterilization of the bottling obtained according to step (c) in a conventional manner.

2. Formulation according to claim 1, **characterized in that** the content of other radionuclides, especially the content of the radionuclides which cited below, does not exceed the indicated numerical value for each:
| radionuclide | max. concentration (mBq/g) |
|---|---|
| ²²⁸Ra | 26 |
| ²²⁸Th | 3.6 |
| ²¹⁰Pb | 23 |
| ²²⁶Ra | 29 |
| ²³⁸U | 19 |
| ²³¹Pa | 55 |
| ²²⁷Ac | 9.3 |
| ²³²U | 20 |
produced by a process which comprises the process steps (a) to (d) described below:
(a) at least one-time centrifugation of an aqueous suspension of the compound ²²⁸Th (OH)₄, optionally after resuspension of the resulting raw sediment;
(b) separation of the ²²⁸Th(OH)₄ sediment which was precipitated according to step (a);
(c) sterile filtration and subsequent bottling of the desired dose of the supernatant solution of the ²²⁴Ra salt which was obtained according to step (b);
(d) sterilization of the bottling obtained according to step (c) in a conventional manner.

3. Formulation according to claim 1, **characterized in that** the content of other radionuclides, especially the content of the radionuclides cited below, does not exceed the indicated numerical value for each:
| radionuclide | max. concentration (mBq/g) |
|---|---|
| ²²⁸Ra | 22 |
| ²²⁸Th | 3.3 |
| ²¹⁰Pb | 17 |
| ²²⁶Ra | 26 |
| ²³⁸U | 15 |
| ²³¹Pa | 55 |
| ²²⁷Ac | 8.4 |
| ²³²U | 5 |
produced by a process which comprises the process steps (a) to (d) described below:
(a) at least one-time centrifugation of an aqueous suspension of the compound ²²⁸Th(OH)₄, optionally after resuspension of the resulting raw sediment;
(b) separation of the ²²⁸Th(OH)₄ sediment which was precipitated according to step (a);
(c) sterile filtration and subsequent bottling of the desired dose of the supernatant solution of the ²²⁴Ra salt which was obtained according to step (b);
(d) sterilization of the bottling obtained according to step (c) in a conventional manner.

4. Formulation according to any one of claims 1 to 3, **characterized in that** the salt of the radionuclide ²²⁴Ra is ²²⁴radium chloride (²²⁴RaCl₂).

5. Formulation according to any one of claims 1 to 4, **characterized in that** the formulation comprises an isotonic agent, preferably a calcium salt, especially calcium chloride.

6. Process for producing a radiotherapeutic formulation which comprises at least one salt of the isotope ²²⁴Ra, **characterized in that** the process steps (a) to (d) which are described below are carried out:
(a) at least one-time centrifugation of an aqueous suspension of the compound ²²⁸Th(OH)₄, optionally after resuspension of the resulting raw sediment;
(b) separation of the ²²⁸Th(OH)₄ sediment which was precipitated according to step (a);
(c) sterile filtration and subsequent bottling of the desired dose of the supernatant solution of the ²²⁴Ra salt which was obtained according to step (b);
(d) sterilization of the bottling obtained according to step (c) in a conventional manner.

7. Process according to claim 6, **characterized in that** the centrifugation in step (a) is carried out twice and the resulting precipitate is resuspended afterwards in each case using an aqueous solution of an isotonic, preferably a calcium salt, especially a 1.14% by weight CaCl₂ solution.

8. Process according to any one of claims 6 or 7, **characterized in that** centrifugation in step (a) is carried out with a rotational speed corresponding to a centrifugal acceleration of 2000 g over a period of 10 minutes.

9. Process according to any one of claims 6 to 8, **characterized in that** separation in step (b) is carried out by suction and filtration.

10. Process according to any one of claims 6 to 9, **characterized in that** sterile filtration in step (c) is carried out using a known sterile filter material, especially by using a material based on a fluorine-containing organic commodity polymer or an optionally modified and/or derivatized polysaccharide.

11. Process according to any one of claims 6 to 10, **characterized in that** the sterile filter material is cellulose, cellulose acetate or polytetrafluoroethylene, preferably cellulose acetate.

12. Process according to any one of claims 6 to 11, **characterized in that** the sterile filter material has a pore size in the range from about 0.01 µm to 10 µm, preferably in the range from about 0.2 µm to 5 µm and in particular about 0.2 µm.

13. Process according to any one of claims 6 to 12, **characterized in that** the sterilization step (d) is carried out using a conventional autoclave, preferably at a temperature of about 121°C over a period of about 20 minutes.

14. Process according to any one of claims 6 to 13, **characterized in that** the therapeutically effective salt of the radionuclide ²²⁴ Ra is ²²⁴radium chloride (²²⁴RaCl₂).

15. Use of a formulation according to any one of claims 1 to 5 for the preparation of a medicament for treatment of ankylosing spondylitis (Bechterew's disease).

## Revendications

1. Formulation radiothérapeutique efficace contenant au moins un sel de l'isotope ²²⁴Ra et **caractérisée en ce que** la teneur en autres radionucléides, notamment la teneur en radionucléides suivants, ne dépasse pas les valeurs numériques respectives indiquées :
| Radionucléide : | Concentration maximale (mBq/g) |
|---|---|
| ²²⁸Ra | 60 |
| ²²⁸Th | 30 |
| ²¹⁰Pb | 30 |
| ²²⁶Ra | 30 |
| ²³⁸U | 20 |
| ²³¹Pa | 60 |
| ²²⁷Ac | 10 |
| ²³²U | 50, |
produite suivant un procédé comprenant les étapes (a) à (d) décrites ci-après :
(a) au moins une centrifugation d'une suspension aqueuse du composé ²²⁸Th(OH)₄, éventuellement après une remise en suspension du sédiment brut obtenu ;
(b) séparation du sédiment de ²²⁸Th(OH)₄ précipité à l'issue de l'étape (a) ;
(c) filtration stérile puis remplissage de la dose souhaitée de la solution d'un sel de ²²⁴Ra surnageante obtenue à l'issue de l'étape (b) ;
(d) stérilisation, de manière connue en soi, de la solution de remplissage obtenue à l'issue de l'étape (c).

2. Formulation selon la revendication 1, **caractérisée en ce que** la teneur en autres radionucléides, notamment la teneur en radionucléides suivants, ne dépasse pas les valeurs numériques respectives indiquées :
| Radionucléide : | Concentration maximale (mBq/g) |
|---|---|
| ²²⁸Ra | 26 |
| ²²⁸Th | 3,6 |
| ²¹⁰Pb | 23 |
| ²²⁶Ra | 29 |
| ²³⁸U | 19 |
| ²³¹Pa | 55 |
| ²²⁷Ac | 9,3 |
| ²³²U | 20, |
produite suivant un procédé comprenant les étapes (a) à (d) décrites ci-après :
(a) au moins une centrifugation d'une suspension aqueuse du composé ²²⁸Th(OH)₄, éventuellement après une remise en suspension du sédiment brut obtenu ;
(b) séparation du sédiment de ²²⁸Th(OH)₄ précipité à l'issue de l'étape (a) ;
(c) filtration stérile puis remplissage de la dose souhaitée de la solution d'un sel de ²²⁴Ra surnageante obtenue à l'issue de l'étape (b) ;
(d) stérilisation, de manière connue en soi, de la solution de remplissage obtenue à l'issue de l'étape (c).

3. Formulation selon la revendication 1, **caractérisée en ce que** la teneur en autres radionucléides, notamment la teneur en radionucléides suivants, ne dépasse pas les valeurs numériques respectives indiquées :
| Radionucléide : | Concentration maximale (mBq/g) |
|---|---|
| ²²⁸Ra | 22 |
| ²²⁸Th | 3,3 |
| ²¹⁰Pb | 17 |
| ²²⁶Ra | 26 |
| ²³⁸U | 15 |
| ²³¹Pa | 55 |
| ²²⁷Ac | 8,4 |
| ²³²U | 5, |
produite suivant un procédé comprenant les étapes (a) à (d) décrites ci-après :
(a) au moins une centrifugation d'une suspension aqueuse du composé ²²⁸Th(OH)₄, éventuellement après une remise en suspension du sédiment brut obtenu ;
(b) séparation du sédiment de ²²⁸Th(OH)₄ précipité à l'issue de l'étape (a) ;
(c) filtration stérile puis remplissage de la dose souhaitée de la solution d'un sel de ²²⁴Ra surnageante obtenue à l'issue de l'étape (b) ;
(d) stérilisation, de manière connue en soi, de la solution de remplissage obtenue à l'issue de l'étape (c).

4. Formulation selon l'une des revendications 1 à 3, **caractérisée en ce que** le sel du radionucléide ²²⁴Ra est du chlorure de ²²⁴radium (²²⁴RaCl₂).

5. Formulation selon l'une des revendications 1 à 4, **caractérisée en ce que** la formulation comprend un isotonique, de préférence un sel de calcium, notamment le chlorure de calcium.

6. Procédé de production d'une formulation radiothérapeutique comprenant au moins un sel de l'isotope ²²⁴Ra, **caractérisé en ce qu'**on conduit les étapes de procédé (a) à (d) suivantes :
(a) au moins une centrifugation d'une suspension aqueuse du composé ²²⁸Th(OH)₄, éventuellement après une remise en suspension du sédiment brut obtenu ;
(b) séparation du sédiment de ²²⁸Th(OH)₄ précipité à l'issue de l'étape (a) ;
(c) filtration stérile puis remplissage de la dose souhaitée de la solution d'un sel de ²²⁴Ra surnageante obtenue à l'issue de l'étape (b) ;
(d) stérilisation, de manière connue en soi, de la solution de remplissage obtenue à l'issue de l'étape (c).

7. Procédé selon la revendication 6, **caractérisé en ce qu'**on conduit deux fois la centrifugation de l'étape (a), puis on remet en suspension le précipité obtenu en utilisant respectivement une solution aqueuse d'un isotonique, de préférence un sel de calcium, notamment une solution à 1,14 pour cent en poids de CaCl₂.

8. Procédé selon l'une des revendications 6 ou 7, **caractérisé en ce qu'**on conduit la centrifugation de l'étape (a) à une vitesse de rotation correspondant à une accélération centrifuge de 2000 g pendant une période de 10 minutes.

9. Procédé selon l'une des revendications 6 à 8, **caractérisé en ce qu'**on conduit la séparation de l'étape (b) par aspiration et filtration.

10. Procédé selon l'une des revendications 6 à 9, **caractérisé en ce qu'**on conduit la filtration stérile de l'étape (c) en utilisant un matériau filtrant stérile connu, de préférence en utilisant un matériau à base de polymère usuel organique fluoré ou d'un polysaccharide éventuellement modifié et/ou dérivé.

11. Procédé selon l'une des revendications 6 à 10, **caractérisé en ce que** le matériau filtrant stérile est de la cellulose, de l'acétate de cellulose ou du polytétrafluoroéthylène, de préférence de l'acétate de cellulose.

12. Procédé selon l'une des revendications 6 à 11, **caractérisé en ce que** le matériau filtrant stérile présente des pores d'une taille comprise dans la plage allant d'environ 0,01 µm à 10 µm, de préférence dans la plage allant d'environ 0,2 µm à 5 µm et notamment d'environ 0,2 µm.

13. Procédé selon l'une des revendications 6 à 12, **caractérisé en ce qu'**on conduit l'étape de stérilisation (d) en utilisant un autoclave usuel, de préférence à une température d'environ 121 °C pendant une période d'environ 20 minutes.

14. Procédé selon l'une des revendications 6 à 13, **caractérisé en ce que** le sel du radionucléide ²²⁴Ra à action thérapeutique est du chlorure de ²²⁴radium (²²⁴RaCl₂).

15. Utilisation d'une formulation selon l'une des revendications 1 à 5 pour produire un médicament destiné à la thérapie de la spondylite ankylosante (maladie de Bechterew).
